# FASCICULE DE BREVET EUROPEEN

(11) **EP 0 826 068 B1**
(45) Date de publication et mention de la délivrance du brevet: **31.05.2000**
(21) Numéro de dépôt: 96914258.7
(22) Date de dépôt: 29.04.1996
(51) Int. Cl.: C12Q 1/68, C07H 21/04, C12P 19/34

(54) **METHODE DE DIAGNOSTIC DE LA SCHIZOPHRENIE**
VERFAHREN ZUR DIAGNOSE VON SCHIZOPHRENIE
METHOD FOR DIAGNOSING SCHIZOPHRENIA

(30) Priorité: 03.05.1995 FR 9505264
(43) Date de publication de la demande: 04.03.1998
(73) Titulaire: Aventis Pharma S.A., 92165 Antony Cédex (FR)
(72) Inventeur: LAURENT, Claudine, 92210 Saint-Cloud (FR); MALLET, Jacques, 75013 Paris (FR); MELONI, Rolando, 75009 Paris (FR)
(86) Numéro de dépôt international: FR9600650
(87) Numéro de publication internationale: WO9634980

(56) Documents cités:
- WO-A-94/03640
- C.R. ACAD SCI SER III, vol. 318, no. 7, Juillet 1995, pages 803-9, XP002012400 MELONI, R. ET AL.: "a rare allele of a microsatellite located in the tyrosine hydroxylase gene found in schizophrenic patients"
- THE LANCET, vol. 345, Avril 1995, page 932 XP002012401 MELONI, R. ET AL.: "association of maniac-depressive illness with tyrosine hydroxylase microsatellite marker."
- GENOMICS , vol. 12, 1992, pages 241-53, XP002012402 EDWARDS, A. ET AL: "genetic variation at five trimeric and tetrameric tandem repeat loci in four human populations" cité dans la demande
- PSYCHIATRIC GENETICS, vol. 3, no. 1, 1993, pages 29-31, XP002012403 BYERLEY, W. ET AL: "tyrosine hydroxylase gene not linked to schizophrenia in nine pedigrees"
- PSYCHIATRIC GENETICS, vol. 5, no. 2, 1995, pages 83-88, XP002012404 WEI, J. ET AL: "association of polymorphic VNTR region in the first intron of the human TH gene with disturbances of the catacholamine pathway in schizophrenia"

### LISTE DE SEQUENCES

(1) INFORMATIONS GENERALES:
   (i) DEPOSANT:
      (A) NOM: RHONE-POULENC RORER S.A.
      (B) RUE: 20, avenue Raymond ARON
      (C) VILLE: ANTONY
      (E) PAYS: FRANCE
      (F) CODE POSTAL: 92165
   (ii) TITRE DE L'INVENTION: METHODE DE DIAGNOSTIC DE LA SCHIZOPHRENIE
   (iii) NOMBRE DE SEQUENCES: 7
   (iv) FORME DECHIFFRABLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 18 paires de bases
      (B) TYPE: nucléotide
      (C) NOMBRE DE BRINS: double
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 1:
   GGCAAATAGG GGGCAAAA 18
(2) INFORMATIONS POUR LA SEQ ID NO: 2:
   (i) CARACTERISTIQUES DE LA SEQUENCE:
      (A) LONGUEUR: 18 paires de bases
      (B) TYPE: nucléotide
      (C) NOMBRE DE BRINS: double
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 2:
   TTATCCAGCC TGGCCCAC 18
(2) INFORMATIONS POUR LA SEQ ID NO: 3:
   (i) CARACTERISTIQUES DE LA SEQUENCE:
      (A) LONGUEUR: 18 paires de bases
      (B) TYPE: nucléotide
      (C) NOMBRE DE BRINS: double
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 3:
   GGCAAATAGG GGGCAAAA 18
(2) INFORMATIONS POUR LA SEQ ID NO: 4:
   (i) CARACTERISTIQUES DE LA SEQUENCE:
      (A) LONGUEUR: 18 paires de bases
      (B) TYPE: nucléotide
      (C) NOMBRE DE BRINS: double
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 4:
   GGCTTCCGAG TGCAGGTC 18
(2) INFORMATIONS POUR LA SEQ ID NO: 5:
   (i) CARACTERISTIQUES DE LA SEQUENCE:
      (A) LONGUEUR: 18 paires de bases
      (B) TYPE: nucléotide
      (C) NOMBRE DE BRINS: double
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 5:
   GTTCCTCCCT TATTTCCC 18
(2) INFORMATIONS POUR LA SEQ ID NO: 6:
   (i) CARACTERISTIQUES DE LA SEQUENCE:
      (A) LONGUEUR: 18 paires de bases
      (B) TYPE: nucléotide
      (C) NOMBRE DE BRINS: double
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 6:
   AGGGAACACA GACTCCAT 18
(2) INFORMATIONS POUR LA SEQ ID NO: 7:
   (i) CARACTERISTIQUES DE LA SEQUENCE:
      (A) LONGUEUR: 636 paires de bases
      (B) TYPE: nucléotide
      (C) NOMBRE DE BRINS: double
      (D) CONFIGURATION: linéaire
   (ii) TYPE DE MOLECULE: ADNc
   (iii) HYPOTHETIQUE: NON
   (iv) ANTI-SENS: NON
   (xi) DESCRIPTION DE LA SEQUENCE: SEQ ID NO: 7:
   CTTGAATCTT AACGATCGGA ATGTGGAAAC AAATCCATCC AAAAAATCCA AGATGGCCAG 60
   AGGTCCCCGG CTGCTGCACC CAGCCCCCAC CCTACTCCCA CCTGCCCCTG CCTCCCTCTG 120
   CCCCAGCTGC CCTAGTCAGC ACCCCAACCA GCCTGCCTGC TTGGGGAGGC AGCCCCAAGG 180
   CCCTTCCCAG GCTCTAGCAG CAGCTCATGG TGGGGGGTCC TGGGCAAATA GGGGGCAAAA 240
   TTCAAAGGGT ATCTGGGCTC TGGGGTGATT CCCATTGGCC TGTTCCTCCC TTATTTCCCT 300
   CATTCATTCA TTCATTCATT CATTCATTCA TTCATTCACC ATGGAGTCTG TGTTCCCTGT 360
   GACCTGCACT CGGAAGCCCT GTGTACAGGG GACTGTGTGG GCCAGGCTGG ATAATCGGGA 420
   GCTTTTCAGC CCACAGGAGG GGTCTTCGGT GCCTCCTTGG GCACTCAGAA CCTTGGGCTC 480
   CCTGGCACAT TTAAAATGGG TTTTTATTTA TGGACCTTGA TTGAAATGTG GTGTGAGTTG 540
   TAGCAGTGTC ATTTCCAGGT ACCTTCTCAG GGACACAGGG CGCCCTCCCC CGTCCTCCCC 600
   CGCCCTCCCC TACCCTCCCC CACCAGGCTC CCCATC 636

## Description

La présente invention concerne une méthode de diagnostic de la schizophrénie. Elle concerne plus particulièrement un procédé de détection de variations dans une séquence répétée d'ADN présente dans le gène TH, dont certaines formes apparaissent spécifiquement dans les patients schizophréniques. L'invention concerne également des amorces utilisables pour le détection d'allèles spécifiques de cette séquence répétée, associées à la schizophrénie.

La présence de mutations dans toutes les classes de séquences répétées d'ADN est un phénomène connu. Cependant, la signification fonctionnelle de ces mutations est indéterminée. Ainsi, les microsatellites représentent une classe abondante de séquences répétées d'ADN qui présentent un grand polymorphisme lié à des variations dans le nombre de motifs répétés (ref 1) et/ou dans la séquence de ces motifs. Ces microsatellites ont pour cette raison été utilisés comme marqueurs génétiques pour la construction de cartes génétiques et pour l'identification de loci impliqués dans des pathologies (ref 2). La taille des différents allèles d'un microsatellite dépend de variations dans le nombre de motifs répétés. Des expériences de séquençage de dimères répétés ont ainsi permis de montrer une variation dans le nombre de motifs répétés et dans leur séquence. Ces variations peuvent correspondre notamment à des répétitions "parfaites", c'est-à-dire sans interruption dans la séquence de bases, ou à des répétitions "imparfaites", contenant une ou plusieurs interruptions dans la séquences des motifs, qu'il s'agisse de délétion(s) ou d'insertion(s) (ref 3). De la même façon, des variations de longueur et/ou de séquence ont également été observées dans les motifs trimériques ou tétramériques répétés. Ainsi, des variations de ce type ont été observées dans les microsatellites HUMHPRTB (ref 4) et HUMTH01 (ref 5).

La demanderesse s'est intéressée plus particulièrement à la recherche d'altérations génétiques liées à la schizophrénie. A cet effet, la demanderesse a réalisé une étude d'association entre le gène de la tyrosine hydroxylase (TH) et la schizophrénie, orientée plus particulièrement sur le microsatellite HUMTH01. La TH est l'enzyme limitante de la voie de biosynthèse des catécholamines. Différentes études génétiques des pathologies psychiatriques et neurologiques ont été réalisées utilisant des marqueurs localisés dans le gène TH (ref 6 et 7). Cependant, jusqu'à présent, il n'existe dans la littérature aucune démonstration d'un association génétique avec la schizophrénie.

Le microsatellite HUMTH01 est localisé dans le premier intron du gène de la tyrosine hydroxylase. Ce microsatellite est constitué de motifs tétramériques TCAT répétés. Il présente un certain polymorphisme, différents allèles ayant été décrits possédant des nombres de motifs répétés variables. L'allèle le plus souvent rencontré comporte 10 motifs répétés et une délétion d'une paire de base dans le cinquième motif répété, qui comporte la séquence CAT.

La demanderesse a ainsi examiné l'implication du gène TH dans la schizophrénie, en recherchant la présence de variations de séquence et de longueur dans le microsatellite HUMTH01. Les résultats obtenus ont permis de montrer que l'allèle parfait était très rare, et présent uniquement chez les patients schizophrènes. Ces résultats ont été reproduits sur des populations de patients d'origine ethnique différente. Ainsi, sur une population française de 239 sujets dont 94 malades de schizophrénie, 6 allèles différents, désignés A, B, C, D, Ei et Ep, ont été détectés. Ces différents allèles diffèrent les uns des autres de 4 paires de bases (1 motif complet), à l'exception des deux plus longs qui diffèrent d'une seule paire de bases. Le séquençage de ces différents allèles a permis de montrer que le motif répété du microsatellite HUMTH01, de séquence TCAT, est parfaitement répété dans les allèles A, B, C et D, qui contiennent respectivement 6, 7, 8 et 9 motifs répétés. En revanche l'allèle E, qui comporte 10 motifs répétés, présente dans la majorité des cas la même délétion d'une thymidine dans le cinquième motif répété. Cette délétion conduit à un allèle imparfait de séquence (TCAT)4(CAT)(TCAT)5, désigné Ei (pour imparfait). L'allèle Ei est l'allèle le plus fréquent dans la population caucasienne (ref 5). Au contraire, l'allèle E parfait de séquence (TCAT)10 (désigné Ep) est très rare. Ainsi, sur l'ensemble de la population de schizophrènes testée, seuls 5 patients possédaient l'allèle parfait. Les résultats obtenus montrent de manière inattendue que tous les sujets portant l'allèle Ep sont des patients schizophrènes, alors que cet allèle est absent de 145 témoins sains (Cf table 1). Ces résultats démontrent une association hautement significative entre la présence de l'allèle Ep et la schizophrénie. En outre les 5 patients schizophrènes porteurs de l'allèle Ep sont des cas sporadiques de schizophrénie dont le subtype clinique est une schizophrénie paranoïde dans un cas, une schizophrénie indifferenciée dans 3 cas et une schizophrénie désorganisée dans 1 cas.

La demanderesse a ensuite étendu cette étude à une autre population d'origine ethnique différente. Ainsi, une étude d'association similaire a été entreprise sur une population de 88 tunisiens. Les résultats obtenus montrent que la fréquence des différents allèles A-E rencontrés dans la population tunisienne testée est significativement différente de celle observée dans la population française (Cf tableau 1). Pourtant, seulement 4 individus se sont avérés être porteurs de l'allèle parfait Ep, tous étant des patients schizophrènes (1 schizophrénie paranoïde et 3 schizophrénies indifférenciées) les formes étant là aussi des formes sporadiques.

Ces résultats constituent la première démonstration d'une association entre la TH et la schizophrénie. Ils montrent clairement que l'allèle parfait Ep du microsatellite HUMTH01 de séquence (TCAT)10 peut être associé de manière significative à la schizophrénie et constitue de ce fait un outil génétique de dépistage de ce type de pathologie.

La spécificité de l'association de cet allèle à la schizophrénie a en outre été confirmée par une étude sur une population de 100 patients atteints de psychose maniaco-dépressive sporadique. La présence de l'allèle Ep n'a été détectée dans aucun de ces patients.

La mise en évidence de l'association entre cet allèle et la schizophrénie offre de nombreuses applications, dans le domaine diagnostique et thérapeutique. Ainsi, la présente invention offre maintenant, pour la première fois, la possibilité de diagnostiquer la schizophrénie par un test biologique et non plus exclusivement par des arguments cliniques. Un objet de la présente invention réside plus particulièrement dans une méthode de diagnostic de la schizophrénie consistant à détecter la présence de l'allèle Ep du microsatellite HUMTH01 dans le gène TH. Cette méthode est également applicable au diagnostic des pathologies du spectre de la schizophrénie, telles que notamment la schizotypie, les individus schizoïdes, etc. L'invention permet ainsi d'affiner les critères de diagnostic de ces pathologies, qui sont aujourd'hui uniquement de nature clinique. En outre, l'invention permet également de mettre en évidence des prédispositions à ce type de trouble psychiatrique, par identification d'une vulnérabilité génétique dans les familles de patients porteurs de cet allèle Ep. D'un point de vue thérapeutique, l'invention permet de manière avantageuse de définir des traitements médicaux plus appropriés en fonction du type de schizophrénie. En confirmant l'hypothèse d'une implication de la voie des catécholamines, l'invention permet d'utiliser des thérapies plus ciblées. En outre, si les implications fonctionnelles de la présence de l'allèle Ep ne sont pas définitivement établies, il est à noter que le microsatellite HUMTH01 est localisé dans le premier intron du gène TH, région dans laquelle il a été démontré un épissage alternatif conduisant à 4 isoformes de la TH (ref 8). Il est donc possible que des variations génétiques dans cette région affectent la régulation de l'expression du gène TH, et que cela soit lié à l'apparition de la schizophrénie.

Selon un premier aspect, l'invention concerne donc une méthode de diagnostic de la schizophrénie caractérisée par la détection in vitro de la présence de l'allèle Ep du microsatellite HUMTH01 dans le gène TH. La mise en évidence de cet allèle, de séquence (TCAT)10, est caractéristique de certaines schizophrénies. L'absence de cet allèle n'exclut pas l'existence d'une schizophrénie, cet allèle étant rencontré dans 5% des cas environ. Le procédé de l'invention est également applicable à la caractérisation génétique de schizophrénies, et au sous-typage de schizophrénie. Comme indiqué précédemment, l'allèle Ep ne semble présent que dans des schizophrènies sporadiques. Le procédé de l'invention est également applicable la mise en évidence de prédisposition à la schizophrénie.

Dans le procédé de l'invention, la détection de l'allèle Ep peut être réalisée par différentes techniques. Parmi les techniques utilisables, on peut citer préférentiellement le séquençage, la séparation sur gel ou encore la technique de SSCP ("single strand conformation polymorphism").

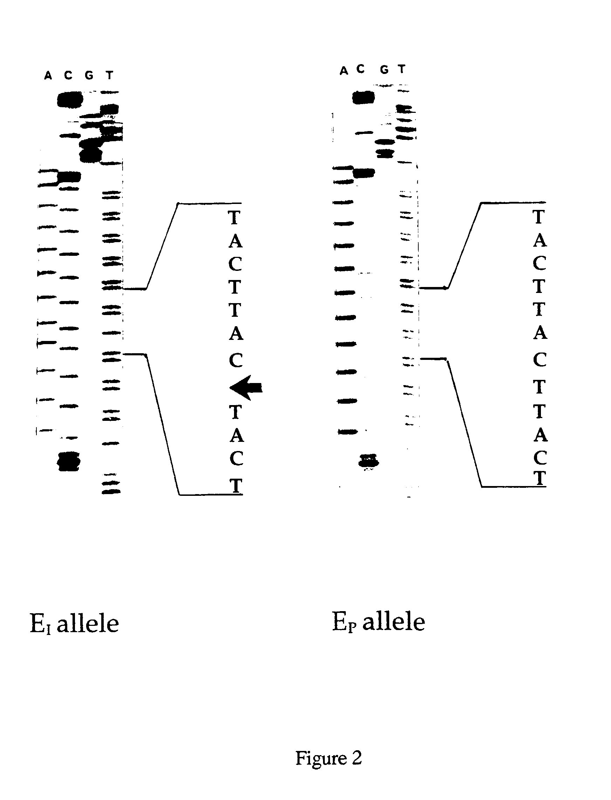

Concernant le séquençage, toute méthode connue de l'homme du métier peut être utilisée. Notamment, il est avantageux d'utiliser un séquenceur automatique d'ADN. Le séquencage est préférentiellement éffectué sur des matrices double-brin par la méthode de terminaison de chaînes en utilisant des amorces fluorescentes. Un kit approprié à cet effet est le kit de séquencage Taq Dye Primer Kit de chez Applied Biosystem (Applied Biosystem, Foster City CA). Le séquençage du microsatellite HUMTH01, ou plus précisément d'un fragment isolé portant ce microsatellite, permet directement de connaître l'allèle présent chez le patient et ainsi de mettre en évidence la présence ou non de l'allèle Ep de séquence (TCAT)10. Les résultats obtenus par séquençage sont présentés sur la figure 2 par exemple.

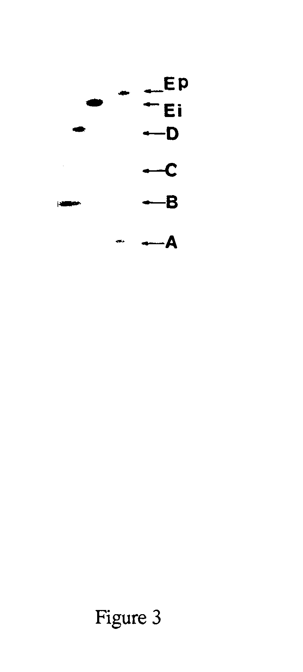

Une technique préférée pour la mise en évidence de l'allèle Ep est la séparation sur gel. Cette technique présente l'avantage de permettre la discrimination entre les différents allèles en fonction de leur taille, sans qu'il soit nécessaire de séquencer les fragments d'ADN. Cette technique repose sur la migration, en condition dénaturante, des fragments d'ADN dénaturés dans un gel d'acrylamide (de préférence à 6%). La révélation des bandes peut être réalisée par toute technique connue de l'homme du métier, basée par exemple sur l'emploi d'amorces marquées (notamment en γ sur le phosphore), sur l'introduction d'α-dCTP dans les fragments testés, sur l'emploi α (alpha) de sondes froides, sur une révélation au bromure d'éthidium, ou encore par hybridation (blot) avec une sonde radiomarquée. Un protocole précis de séparation sur gel est donné à titre illustratif dans les exemples. Comme indiqué sur la figure 3, cette technique permet, rapidement et sans séquençage, de distinguer entre les allèles A, B, C, D, Ei et Ep du microsatellite.

Concernant la technique d'identification par SSCP, il s'agit également d'une méthode de séparation sur gel d'acrylamide, mais en condition non dénaturante. Cette technique permet de discriminer les différents fragments en fonction de leur conformation (Cf exemples).

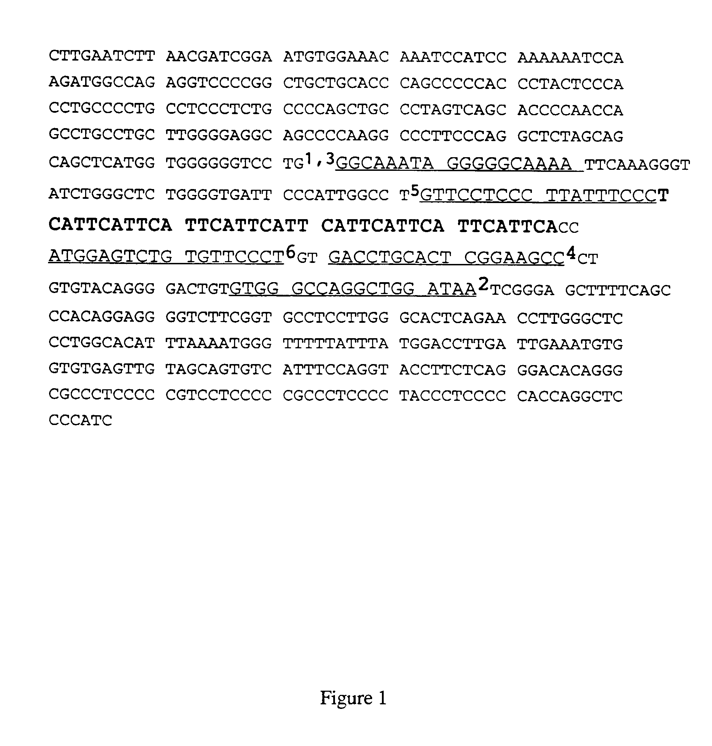

Le procédé de l'invention est réalisé sur un échantillon d'ADN du patient. Cet échantillon doit contenir au moins le microsatellite HUMTH01. Il contient préférentiellement tout ou partie du premier intron du gène TH. Encore plus préférentiellement, il comporte un fragment du gène TH contenant le microsatellite HUMTH01 bordé de séquences flanquantes. L'échantillon d'ADN à tester peut être obtenu à partir de cellules prélevées au patient. Il s'agit préférentiellement de cellules sanguines (cellules mononucléées par exemple), qui sont aisément obtenues par simple prélèvement sanguin. D'autres types cellulaires peuvent être utilisés tels que notamment les fibroblastes, les cellules épithéliales, les kératinocytes, etc. L'ADN est ensuite extrait des cellules, et utilisé pour la détection de l'allèle Ep. Pour cela, l'ADN génomique obtenu peut être digéré par des enzymes de restriction, cloné dans des vecteurs appropriés, sélectionné pour la TH par exemple ou par hybridation avec une sonde correspondant au premier intron de la TH, puis analysé comme décrit ci-dessus. De manière tout a fait préférée, l'ADN extrait est préalablement soumis à une ou plusieurs réactions d'amplification afin d'obtenir une quantité importante de matériel correspondant à la région portant le microsatellite HUMTH01. L'amplification peut être réalisée par toute technique connue de l'homme du métier, et notamment par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350]. A cet égard, l'amplification peut être effectuée en utilisant un "DNA thermal cycler" (Perkin Elmer Cetus) selon les spécifications du fabricant. Les conditions de température et de milieu utilisées pour l'amplification sont les conditions générales telles que décrites par exemple dans Maniatis et al., 1989. Des conditions spécifiques sont également données dans les exemples. Pour la mise en oeuvre de la présente invention, il est avantageux d'utiliser un fragment d'ADN portant le microsatellite HUMTH01 de taille suffisamment petite. Ceci permet avantageusement une discrimination entre les allèles sans devoir recourir au séquençage. En outre, si des expériences controle de séquençage sont réalisées, il est également préférable de n'avoir à séquencer que des fragments de taille limitée. Avantageusement, le fragment d'ADN utilisé est un fragment amplifié d'une taille inférieure à 300 pb. Ce fragment porte le microsatellite HUMTH01 et des séquences flanquantes du gène TH, telles que représenté sur la figure 1. Encore plus préférentiellement, le fragment amplifié comprend moins de 200pb. Des résultats particulièrement remarquables ont été obtenus avec des fragments amplifiés d'une taille inférieure à 160 pb, et même à 100 pb. A cet effet, la présente invention décrit également des amorces spécifiques permettant l'amplification de fragments d'ADN de petite taille portant le microsatellite HUMTH01. Ainsi, l'invention décrit en particulier 3 couples d'amorces permettant respectivement l'amplification de fragments de 192pb, 156pb et 77pb. La séquence de ces amorces est donnée dans les exemples, ainsi que la séquence et la position sur le gène TH du fragment amplifié. Toute autre amorce permettant l'amplification d'un fragment de moins de 300pb portant au moins le microsatellite HUMTH01 et une région flanquante, dérivée de la séquence présentée sur la figure 1 fait également partie de la présente invention. Avantageusement, les amorces selon l'invention ont une longueur comprise entre 10 et 40 mer et, de préférence, entre 15 et 30 mer. La séquence de ces amorces peut être déterminée à partir de la séquence donnée dans la figure 1, en fonction de la taille du fragment amplifié, de sa localisation autour du microsatellite, et de la longueur choisie des amorces.

Les amorces utilisées dans le cadre de l'invention peuvent être synthétisées selon toute technique connue de l'homme du métier, et notamment en utilisant la chimie des phosphoramidites, éventuellement protégés en β par un groupement cyanoéthyl, (Sinha et al.,1984, Giles 1985), avec un synthétiseur automatique d'ADN tel que le synthétiseur Applied Biosystem modèle 394, (Applied Biosystem, Foster City CA), selon les recommandations du fabricant. Les amorces peuvent également être marquées par toute technique connue de l'homme du métier.

La demande de brevet WO94/03640 décrit un couple d'amorces (SEQ ID NO:62 et SEQ ID NO:63) pouvant permettre l'amplification d'un segment d'ADN génomique contenant le microsatellite HUMTH01.

L'échantillon d'ADN à tester peut être un ADN génomique, un ADNc, ou également un ARN. Il s'agit plus préférentiellement du produit d'amplification de l'ADN génomique amplifié au moyen d'amorces spécifiques telles que décrites ci-avant.

Un autre objet de l'invention concerne une trousse de détection de l'allèle Ep comprenant un couple d'amorces telles que définies ci-avant. Cette trousse est avantageusement une trousse de diagnostic de la schizophrénie.

Comme indiqué précédemment, la présente invention fournit pour la première fois une méthode génétique de détection et de caractérisation génétique des maladies du spectre de la schizophrénie. Outre les applications diagnostic mentionnées, cette méthode offre également des potentialités thérapeutiques importantes, liées notamment à un meilleur ciblage du traitement en fonction de l'affection concernée.

La présente invention sera décrite plus en détail à l'aide des exemples qui suivent, qui doivent être considérés comme illustratifs et non limitatifs.

### Liste des Figures

Figure 1 : Séquence d'une partie du premier intron du gène TH incluant le microsatellite HUMTH01. La position des amorces d'amplification est indiquée.

Figure 2 : Mise en évidence par séquençage de l'allèle Ep du microsatellite HUMTH01.

Figure 3 : Mise en évidence par séparation sur gel de l'allèle Ep du microsatellite HUMTH01.

Table 1 : Distribution des allèles du microsatellite HUMTH01 dans les populations testées atteintes ou non de schizophrénie.

### Exemples

### 1. Préparation de l'ADN

Les échantillons de sang ont été collectés sur héparine et les cellules mononucléées isolées sur gradient Ficoll hypaque (Pharmacia, Upsala, Suède). L'ADN a ensuite été extrait selon les techniques standard. Pour une extraction directe de l'ADN, le protocole suivant a été utilisé :

Le sang récolté a été versé dans des tubes de 50 ml et du tampon de lyse a été ajouté pour faire éclater les hématies (TAMPON LYSE= 40 ml TRIS HCl 1N pH 7.5 + 20 ml EDTA 0,5M + H2O milliQ qsp 2 L), volume final : 50 ml. La suspension est ensuite centrifugée 15 min à 2500 rpm à 4°C; le surnageant est jeté, 50 ml de tampon de lyse sont à nouveau ajoutés au culot. La même opération (centrifugation, élimination du surnageant, reprise du culot dans le tampon de lyse) est répétée deux fois. A l'issue de ces 3 centrifugations, le culot est récupéré.

On ajoute alors :
5 ml de tampon de lyse (pour 5ml de sang au départ)
125 µl de lauryl sarkosyl 20 %
50 µl de proteinase K (20 ng/ml)

La solution obtenue est mélangée et mise au bain marie agitant à 55°C pendant la nuit

Le lendemain matin, 2 volumes d'ethanol 95 % sont ajoutés (si 5 ml → 15 ml terminal), puis la solution est précipitée par inversion jusqu'à la formation d'un flocon. L'ADN est alors récupéré à l'aide d'une pipette P1000 (avec le bout du cône coupé) dans un tube de 5 ml. On ajoute ensuite de l'ethanol 80 % et on place le mélange dans un réfrigérateur. Le lendemain matin l'ethanol est changé puis, le soir, l'alcool est retiré et on laisse sécher le flocon (le tube est posé à l'envers sur un kleenex®).

L'ADN est alors repris avec du TE 1X; suivant la grosseur du flocon la quantité de TE varie de 200 µl à 1 ml. On laisse toute la nuit sur machine rotative à 37°C puis la concentration d'ADN est déterminée par spectrométrie.

### 2. Population française :

94 patients d'origine différente, atteints de schizophrénie chronique (62 hommes et 32 femmes, âge moyen 42+/-12.3) incluant 21 cas familiaux ont été étudiés. Le diagnostic a été formulé selon le critère DSM III (ref 9) en utilisant les données provenant des tableaux médicaux et d'interview directes suivant la traduction française du Tableau des Désordres Affectifs et de l'Anxiété Schizophrénique (SADS-LA, ref 10). A chaque patient a été attribué un sous-type clinique selon la procédure décrite. 145 témoins non apparentés ont été étudiés (84 hommes et 61 femmes; âge moyen 48+/-8.3), ne présentant aucun désordre psychiatrique.

### 3. Population tunisienne :

44 patients d'origine différente, atteints de schizophrénie chronique (33 hommes et 11 femmes, âge moyen 37+/-8.2) incluant 13 cas familiaux ont été étudiés. Le diagnostic a été formulé selon le critère DSM IIIR (ref 11) en utilisant les données provenant des tableaux médicaux. Ces patients ont été comparés à 44 patients contrôle, sans liens entre eux (37 hommes et 7 femmes ; âge moyen 35+/-5.9), ne présentant aucun désordre psychiatrique.

### 4. Amorces utilisées pour les réactions d'amplification

La matrice ciblée pour les réactions d'amplification est composée de l'allèle Ep du microsatellite HUMTH01, dont la séquence est (TCAT)4TCA(TCAT)5. Le microsatellite est localisé à la position 1070 de la séquence du gène TH tel que publiée et accessible dans Genebank (n° D00269).

Différents couples d'amorces ont été utilisées pour l'amplification par PCR. Ces couples sont donnés ci-dessous ainsi que la taille du fragment amplifié. La position de ces amorces sur la séquence du gène TH est donnée dans la figure 1 (voir également SEQ ID n° 1).
Couple A: et
   La longueur d'amplification prévue est de 192 pb.
Couple B: et
   La longueur d'amplification prévue est de 156 pb.
Couple C: et
   La longueur d'amplification prévue est de 77 bp

### 5. Protocole des réactions d'amplification

Le mélange utilisé pour la réaction de PCR contient: 40 ng d'ADN génomique préparé selon l'exemple 1, 200 mM de chaque dATP, dCTP, dGTP et dTTP, 18 pmol (100 ng) de chaque amorce, 50 mM KCl, 10 mMTris-HCl (pH 8,5), 1.5 mM MgCl et 1.0 mCi (a-³²P) dCTP (Amersham, UK) dans un volume final de 15 ml.

Après une dénaturation initiale de 3 min à 96°C, 0.75 unités de Taq Polymérase sont ajoutés à chaque échantillon (ADN+mix) à 92°C, cette étape est suivie par 30 cycles comprenant une d'hybridation pendant 30s à 56°C, une élongation pendant 30s à 72°C et une dénaturation pendant 30s à 92°C.

### 6. Séparation sur gel

Trois microlitres du produit de la réaction de PCR mélangés à 3 microlitres de "stop solution" (0.025 de bleu de bromophénol et 0.025 de xylène cyanol, 0.95 de formamide désionisée) sont chargés dans un gel dénaturant (6% Acrylamide/Bisacrylamide 19:1). Après migration électrophorétique (2500 volts, 55 mA), le gel est démoulé et séché. Il est mis en cassette (sans écran amplificateur) avec un film XOMAT® (Kodak) pour autoradiographie et exposé pendant la nuit à température ambiante.

### 7. Protocole pour la réaction de PCR-SSCP

Cette réaction a été réalisée en plusieurs étapes selon le protocole suivant :
1- Amplification par PCR (Polymerase Chain Reaction) de la séquence cible d'ADN (150-200 pb) avec marquage radioactif des deux brins (utilisation de α ³²P-ATP) ou d'un seul brin (kination d'une des amorces avec γ³²P-ATP).
   Un ml de produit PCR est mélangé avec 2,5 ml de tampon de charge (80 % formamide deionisée, 50 mM TBE, 1 mM EDTA, 0.5 % Xylène-Cyanol, 0.5 % Bleu de Bromo-Phenol).
2- Dénaturation de l'ADN à 96 °C pendant 3-5 min.
3- Refroidissement rapide des échantillons dans la glace fondante.
4- Dépôt rapide dans un gel non-dénaturant de polyacrylamide (6 % Acryl/Bisacrylamide 37.5:1, 0,5X TBE).
5- Migration électrophorétique à 4°C et à 50W ou à température ambiante et à 10W (avec 10 % glycerol dans le gel).
6- Autoradiographie du gel séché.

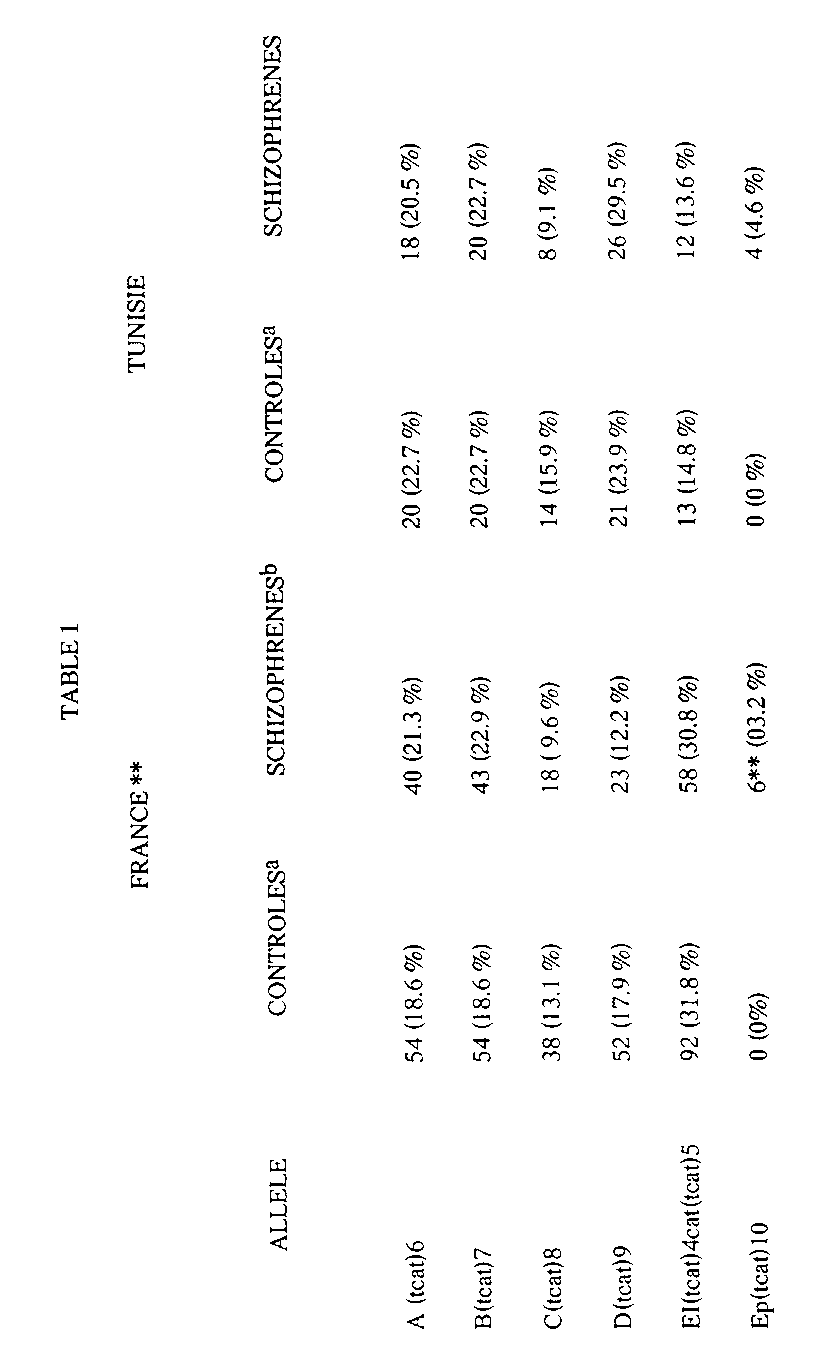

### Liste de Références

1.Weber et al., Am. J. Hum. Genet. 44(1989)388
2. Hearne et al., Trends Genet. 8 (1992) 288
3. Weber et al., Genomics 7 (1990) 524
4. Edwards et al., Genomics 12 (1992) 241
5. Puers et al., AmJ. Hum. Genet. 53 (1993) 953
6. Craddock et al., Ann. of Medecine 25 (1993) 317
7. Leboyer et al., Lancet 335 (1990) 1219
8. Grima et al., Nature 326 (1987) 707
9. American Psychiatric Association, Diagnostic and Statistical Manual of mental disorders (3rd Edn) - Washington, DC = APA (1980).
10. Fyer et al., Anxiety Disorders clinic, New-York : New-York State Psychiatric Institute (1985).
11. American Psychiatric Association, Diagnostic and Statistical Manual of mental disorders (3rd Edn revised) - Washington, DC = APA (1987).

## Revendications

1. Méthode de diagnostic de la schizophrénie caractérisée en ce que l'on détecte in vitro la présence de l'allèle Ep du microsatellitc HUMTH01 dans le gène TH.

2. Méthode selon la revendication 1 caractérisée en ce que la détection de l'allèle parfait Ep est réalisée par séquençage et/ou par séparation sur gel et/ou par SSCP.

3. Méthode selon la revendication 2 caractérisée en ce que la détection de l'allèle parfait Ep est réalisée par séparation sur gel.

4. Méthode selon l'une des revendications précédentes caractérisée en ce que la détection est réalisée sur l'ADN extrait du patient.

5. Méthode selon la revendication 4 caractérisée en ce que l'ADN est extrait à partir de cellules mononucléées.

6. Méthode selon la revendication 4 ou 5 caractérisée en ce que l'ADN est amplifié préalablement à la détection.

7. Méthode selon la revendication 4 caractérisée en ce que l'ADN amplifié comprend tout ou partie du premier intron du gène TH.

8. Méthode selon la revendication 7 caractérisée en ce que l'ADN amplifié est un fragment d'une taille inférieure à 300 pb portant le microsatellite HUMTH01 et des séquences flanquantes.

9. Méthode selon la revendication 8 caractérisée en ce que l'ADN amplifié est un fragment d'une taille inférieure à 200pb et, de préférence, inférieure à 160 pb.

10. Couple d'amorces permettant l'amplification d'un fragment de moins de 200pb portant au moins le microsatellite HUMTH01 et une région flanquante.

11. Couple d'amorces selon la revendication 10 caractérisée en ce que les amorces ont une longueur comprise entre 10 et 40 mer, de préférence entre 15 et 30 mer.

12. Couple d'amorce selon la revendication 11 caractérisée en ce qu'il est choisi parmi le couple A comportant les amorces SEQ ID n° 1 et 2; le couple B comportant les amorces SEQ ID n° 3 et 4 et le couple C comportant les amorces SEQ ID n° 5 et 6.

13. Trousse de détection de l'allèle parfait Ep du microsatellite HUMTH01 comprenant un couple d'amorce selon la revendication 10.

14. Trousse selon la revendication 13 pour le diagnostic de la schizophrénie.

15. Méthode de caractérisation génétique de schizophrénies caractérisée en ce que l'on détecte in vitro la présence de l'allèle parfait Ep du microsatellite HUMTH01 dans le gène TH.

16. Méthode de mise en évidence de prédisposition à la schizophrénie caractérisée en ce que l'on détecte in vitro la présence de l'allèle parfait Ep du microsatellite HUMTH01 dans le gène TH.

## Patentansprüche

1. Verfahren zur Diagnose von Schizophrenie, dadurch gekennzeichnet, dass man in vitro die Anwesenheit des Ep-Allels des Mikrosatelliten HUMTH01 in dem TH-Gen nachweist.

2. Verfahren nach Anspruch 1, dadurch gekennzeichnet, dass man das vollständige Ep-Allel durch Sequenzierung und/oder durch Abtrennung auf Gel und/oder durch SSCP nachweist.

3. Verfahren nach Anspruch 2, dadurch gekennzeichnet, dass man das vollständige Ep-Allel durch Abtrennung auf Gel nachweist.

4. Verfahren nach einem der vorstehenden Ansprüche, dadurch gekennzeichnet, dass man den Nachweis an einem DNA-Extrakt des Patienten durchführt.

5. Verfahren nach Anspruch 4, dadurch gekennzeichent, dass die DNA aus mononucleären Zellen extrahiert wird.

6. Verfahren nach Anspruch 4 oder 5, dadurch gekennzeichent, dass die DNA vor der Detektion amplifiziert wird.

7. Verfahren nach Anspruch 4, dadurch gekennzeichent, dass die amplifizierte DNA die Gesamtheit oder einen Teil des ersten Introns des TH-Gens umfasst.

8. Verfahren nach Anspruch 7, dadurch gekennzeichent, dass die amplifizierte DNA ein Fragment mit einer Größe kleiner als 200 bp ist, das den Mikrosatelliten HUMTH01 und flankierende Sequenzen enthält.

9. Verfahren nach Anspruch 8, dadurch gekennzeichent, dass die amplifizierte DNA ein Fragment mit einer Größe kleiner als 200 bp und bevorzugt kleiner 160 bp ist.

10. Primerpaar, das die Amplifikation eines Fragments von weniger als 200 bp erlaubt, das mindestens den Mikrosatelliten HUMTH01 und eine flankierende Region enthält.

11. Primerpaar nach Anspruch 10, dadurch gekennzeichnet, dass die Primer eine Länge zwischen 10 und 40mer, bevorzugt zwischen 15 und 30mer, besitzen.

12. Primerpaar nach Anspruch 11, dadurch gekennzeichnet, dass es aus dem Paar A, umfassend die Primer SEQ ID NO. 1 und 2, dem Paar B, umfassend die Primer SEQ ID NO. 3 und 4,und dem Paar C, umfassend die Primer SEQ ID NO. 5 und 6, ausgewählt ist.

13. Kit zum Nachweis des vollständigen Ep-Allels des Mikrosatelliten HUMTH01, umfassend ein Primerpaar nach Anspruch 10.

14. Kit nach Anspruch 13 zur Diagnose der Schizophrenie.

15. Verfahren zur genetischen Charakterisierung von Schizophrenen, dadurch gekennzeichnet, dass man in vitro die Anwesenheit des vollständigen Ep-Allels des Mikrosatelliten HUMTH01 in dem TH-Gen nachweist.

16. Verfahren zum Nachweis einer Präsisposition für Schizophrenie, dadurch gekennzeichnet, dass man in vitro die Anwesenheit des vollständigen Ep-Allels des Mikrosatelliten HUMTH01 in dem TH-Gen nachweist.

## Claims

1. Method for diagnosing schizophrenia, characterized in that the presence of the Ep allele of the HUMTH01 microsatellite in the TH gene is detected in vitro.

2. Method according to Claim 1, characterized in that the Ep perfect allele is detected by sequencing and/or by gel separation and/or by SSCP.

3. Method according to Claim 2, characterized in that the Ep perfect allele is detected by gel separation.

4. Method according to one of the preceding claims, characterized in that the detection is effected on DNA which has been extracted from the patient.

5. Method according to Claim 4, characterized in that the DNA is extracted from mononucleated cells.

6. Method according to Claim 4 or 5, characterized in that the DNA is amplified prior to detection.

7. Method according to Claim 4, characterized in that the amplified DNA comprises all or part of the first intron of the TH gene.

8. Method according to Claim 7, characterized in that the amplified DNA is a fragment of less than 300 bp in size, carrying the HUMTH01 microsatellite and flanking sequences.

9. Method according to Claim 8, characterized in that the amplified DNA is a fragment of less than 200 bp in size and, preferably, less than 160 bp in size.

10. Pair of primers which make it possible to amplify a fragment of less than 200 bp carrying at least the HUMTH01 microsatellite and a flanking region.

11. Pair of primers according to Claim 10, characterized in that the primers have a length of between 10 and 40 mer, preferably of between 15 and 30 mer.

12. Pair of primers according to Claim 11, characterized in that it is selected from among the pair A, comprising the primers SEQ ID No. 1 and 2; the pair B, comprising the primers SEQ ID No. 3 and 4, and the pair C, comprising the primers SEQ ID No. 5 and 6.

13. Kit for detecting the Ep perfect allele of the HUMTH01 microsatellite, comprising a pair of primers according to Claim 10.

14. Kit according to Claim 13 for diagnosing schizophrenia.

15. Method for genetically characterizing schizophrenias, characterized in that the presence of the Ep perfect allele of the HUMTH01 microsatellite in the TH gene is detected in vitro.

16. Method for demonstrating predisposition to schizophrenia, characterized in that the presence of the Ep perfect allele of the HUMTH01 microsatellite in the TH gene is detected in vitro.
